# EUROPEAN PATENT SPECIFICATION

(11) **EP 1 836 157 B1**
(45) Date of publication and mention of the grant of the patent: **07.10.2009**
(21) Application number: 06718295.6
(22) Date of filing: 11.01.2006
(51) Int. Cl.: C07C 231/06, C07C 233/06, C07C 209/50, C07C 211/38

(54) **PROCESS FOR THE PREPARATION OF 1-AMINO-3,5-DIMETHYLADAMANTANE HYDROCHLORIDE**
VERFAHREN ZUR HERSTELLUNG VON 1-AMINO-3,5-DIMETHYLADAMANTAN-HYDROCHLORID
PROCEDE DE PREPARATION DU CHLORHYDRATE 1-AMINO-3,5-DIMETHYLADAMANTANE

(30) Priority: 11.01.2005 US 642957 P; 03.05.2005 US 677599 P
(43) Date of publication of application: 26.09.2007
(73) Proprietor: Teva Pharmaceutical Fine Chemicals S.R.L., 23892 Bulciago (IT)
(72) Inventor: MERLI, Valeriano, I-23894 Cremella (IT); MANTOVANI, Silvia, I-20031 Cesano Maderno (IT); DAVERIO, Paola, I-20058 Villasanta (IT); BIANCHI, Stefano, I-22100 Breccia (IT); SPREAFICO, Alessandro, I-23900 Lecco (IT)
(74) Representative: Eder, Michael
(86) International application number: PCT/US2006/001204
(87) International publication number: WO 2006/076562

(56) References cited:
- CZ-B- 282 398
- US-A- 3 391 142
- GERZON K ET AL: "The Adamantyl Group in Medicinal Agents. I. Hypoglycejmic N-Arylsulfonyl-N'-adamantylureas" JOURNAL OF MEDICINAL CHEMISTRY, AMERICAN CHEMICAL SOCIETY. WASHINGTON, US, vol. 6, no. 6, November 1963 (1963-11), pages 760-763, XP002353954 ISSN: 0022-2623

## Description

### FIELD OF THE INVENTION

The present invention is directed to processes for the preparation of 1-amino-3,5-dimethyladamantane hydrochloride, ("memantine hydrochloride") and its intermediates.

### BACKGROUND OF THE INVENTION

Memantine hydrochloride is one of a small group of drugs known as Tricyclic Antivirals (TAVs), and provides good and persistent activation of central nervous system N-methyl-d-aspartate (NMDA) receptors, such that it can be used in the treatment of Parkinson's and Alzheimer's diseases. The chemical structure of memantine hydrochloride is as illustrated in the following scheme.

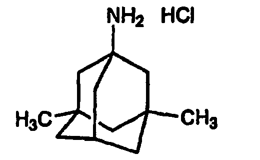

Three strategies for the synthesis of memantine hydrochloride are disclosed in the prior art:
A) N-acetyl-memantine alkaline hydrolysis, disclosed in U.S. Patent No. 3,391,142, Czech Patent Publication No. CZ282398B6, Japanese Patent Publication No. JP 2002 275142A2, and Chinese Patent Publication No. CN 1335299;
B) Reaction of 1-halo-3,5-dimethyladamantane and urea at high temperature, and alkaline hydrolysis of the resulting adamantyl urea intermediate, disclosed in U.S. Patent No. 4,122,193 and Chinese Patent Publication No. CN1400205A; and
C) Electrophylic substitution on metallated 3,5-dimethyladamantane by chloramine, disclosed in U.S. Patent No. 5,599,998.

U.S. Patent No. 3,391,142 discloses the synthesis of memantine hydrochloride and its precursor, n-acetyl-memantine, according to following scheme, where the reported yield of the first step is 100 percent, the reported yield of the second step is 69.8 percent, and the reported overall yield is 69.8 percent.

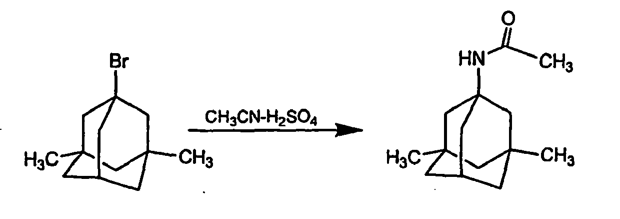

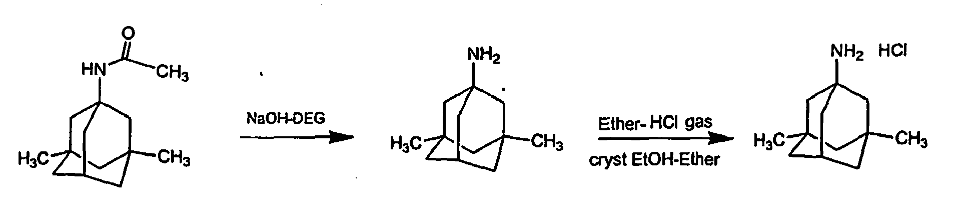

In the first reaction, 1-bromo-3,5-dimethyladamantane reacts with 17 moles of acetonitrile and 35 moles of sulphuric acid at room temperature to give the crude intermediate product in 100 percent yield. The intermediate product is subjected to alkaline hydrolysis with sodium hydroxide in diethylene glycol by refluxing at a temperature of greater than 190°C for six hours. The hydrolyzed product is diluted with water, followed by several benzene extractions, and the memantine free base is recovered by solvent distillation. The free base is then diluted with ether, and the addition of hydrogen chloride gas provides crude memantine hydrochloride. Crystallization of the hydrochloride from an ethanol-ether mixture gives pure memantine hydrochloride in a 69.8 percent yield.

Czech Patent Publication No. CZ282398B6 discloses the synthesis of memantine according to the following scheme, which is carried out under pressure.

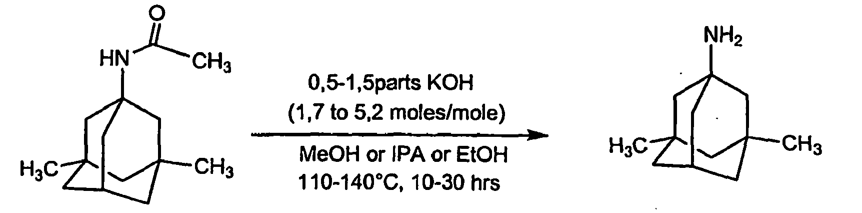

Japanese Patent Publication No. JP 2002 275142A2 discloses the synthesis of n-acetyl-memantine according to the following scheme with a yield of 45 percent.

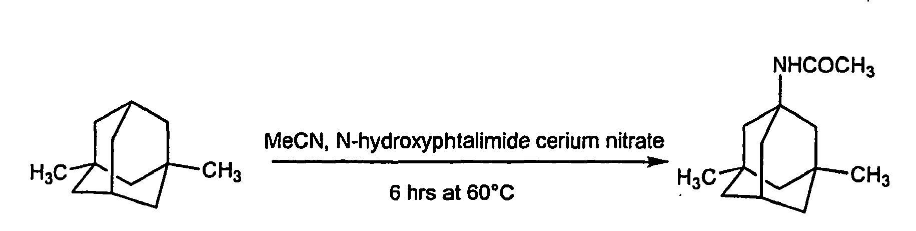

Chinese Patent Publication No. CN 1335299 discloses memantine hydrochloride synthesis according to the scheme.

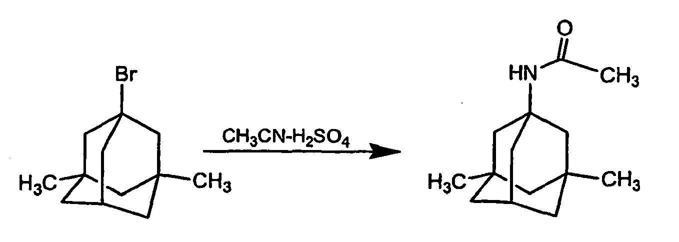

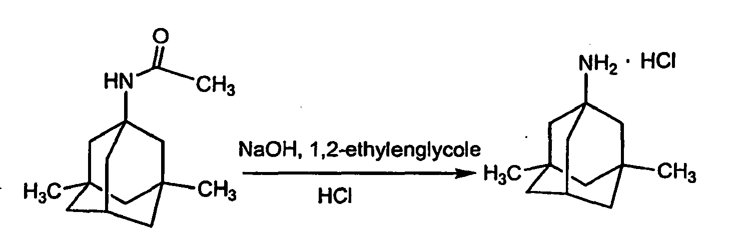

U.S. Patent No. 4,122,193 discloses pharmacological compositions and methods for treating living mammals suffering from hyperkinesias, as well as memantine hydrochloride, hydrobromide, and sulphate synthesis, where the hydrochloride is prepared according to the following scheme with a yield of 78 percent.

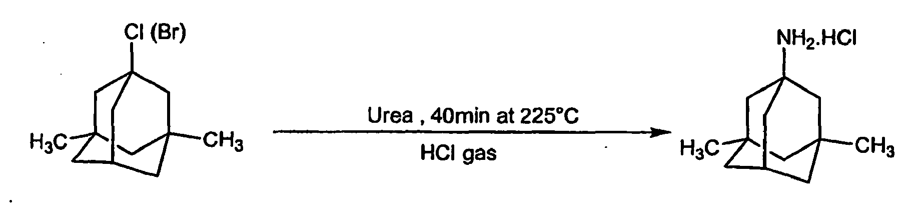

Chinese Patent Publication No. CN1400205A discloses memantine hydrochloride synthesis according to the following scheme.

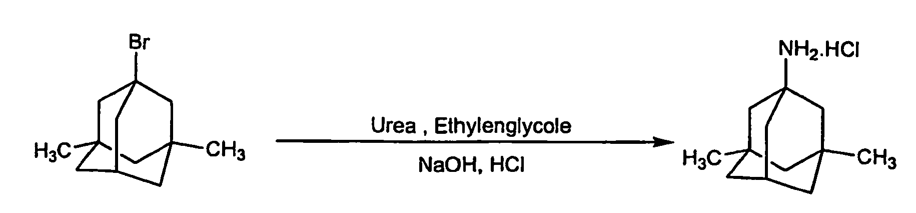

U.S. Patent No. 5,599,998 discloses memantine synthesis with a yield of 48 percent according to the following scheme.

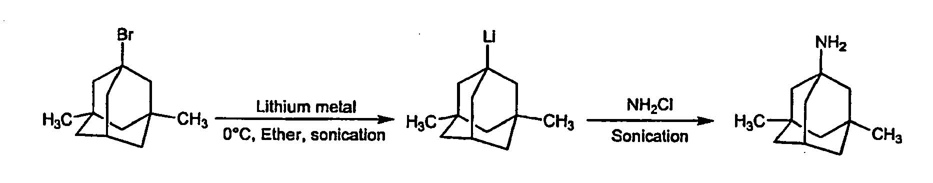

In addition to the low yield, the disclosed method is cumbersome on an industrial scale, using metallic lithium and chloramine, which can be hazardous if not handled properly.

The deficiencies of the prior art processes described above include, but are not limited to the production of undesirable byproducts, such as Br₂ and SO₂, very high reaction temperatures, the need for high pressures, and the use of dangerous reactants. The undesirable byproducts are produced in the Ritter reaction of 1-promo-3,5-dimethyladamantane and acetonitrile in presence of 96 percent sulphuric acid. The very high reaction temperatures, typically from 190° to 225°C are required in the alkaline hydrolysis of n-acetyl-memantine in diethylene glycol or ethylene glycol and in the reaction between 1-halo-3,5-dimethyladamantane and urea. Where the alkaline hydrolysis is performed in low boiling alcohols, elevated pressure is required to attain the reaction temperatures required for an acceptable reaction time. Moreover, as discussed above, the dangerous reactants lithium and chloramine are required in the electrophilic amination reaction. Therefore, there is a need in the art to provide improved processes for preparing memantine hydrochloride.

### SUMMARY OF THE INVENTION

It is an objective of this invention to provide processes for the preparation of memantine, intermediates thereof, and its hydrochloric acid addition salt form without the deficiencies of prior art processes.

In a first embodiment, the present invention is directed to a process for the synthesis of n-acetamid-3,5-dimethyladamantane by reacting 1-halo-3,5-dimethyladamantane and a nitrile, in the presence of phosphoric acid, at a temperature of 50° to 100°C and for a period of time sufficient to produce n-acetamido-3,5-dimethyladamantane, and recovering n-acetamido-3,5-dimethyladamantane.

In a another embodiment, the invention is directed to a process for the synthesis of memantine, comprising: solvolysis of n-acetamido-3,5-dimethyladamantane in a solvent selected from the group consisting of C₄₋₆ alcohols in the presence of a strong inorganic base for a adequate period of time to obtain 1-amino-3,5-dimethyladamantane; and recovering an anhydrous alcoholic solution of 1-amino-3,5-dimethyladamantane.

In a further embodiment, the invention is directed to a "one pot" process for the synthesis of memantine from 1-halo-3,5-dimethyladamantane without the isolation of n-acetyl-3,5-dimethyladamantane.

Preferably, the memantine prepared by the above processes is subsequently converted into a memantine hydrochloride.

Preferably, the memantine prepared by the above processes has a yield which is not less than 80%.

### BRIEF DESCRIPTION OF THE DRAWING

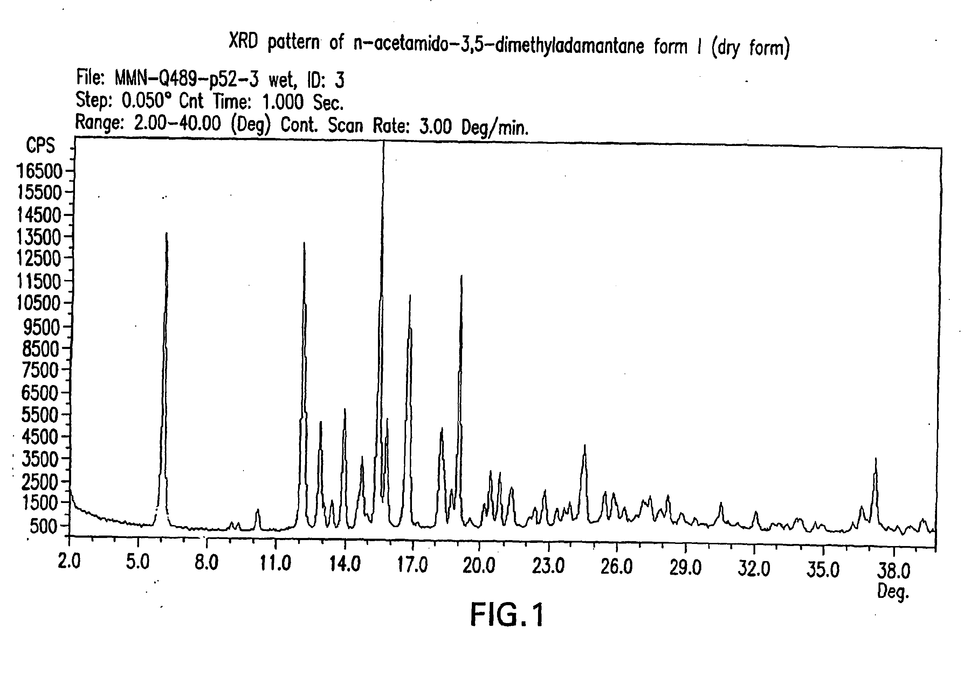

Fig. 1 illustrates a characteristic X-ray powder diffraction pattern of n-acetamido-3,5-dimethyladamantane Form I.

### DESCRIPTION OF THE INVENTION

As used herein, the preface "halo-" refers to chloro, bromo, iodo, and fluoro.

The present invention is directed to processes for preparation of memantine, its intermediates thereof; and its hydrochloric acid salt form that overcome one or more of the problems of the prior art processes. Particularly, the processes may be used to produce the desired product in higher yield than the prior art processes.

In a first embodiment, the present invention is directed to a process for the synthesis of n-acetamido-3,5-dimethyladamantane by providing 1-halo-3,5-dimethyladamantane, as shown below in Scheme 1 and labelled as the 1^{st} step. This process is performed by reacting 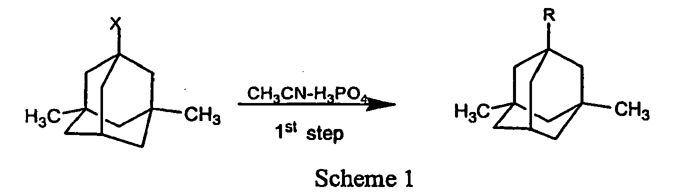 1-halo-3,5-dimethyladamantane and a nitrile, in the presence of phosphoric acid at 50° to 100°C and for a period of time sufficient to produce n-acetamido-3,5-dimethyladamantane, and then recovering n-acetamido-3,5-dimethyladamantane.

Preferably the 1-halo-3,5-dimethyladamantane used is 1-promo-3,5-dimethyladamantane

Preferably, the reaction temperature is 65° to 90°C. An adequate period of time sufficient to produce n-acetamido-3,5-dimethyladamantane is 1 to 20 hours, although most preferably 1 to 10 hours.

The nitrile may be selected from a group consisting of propionitrile, butyronitrile, valeronitrile, phenylacetonitrile, and acetonitrile. Most preferably, acetonitrile is used, preferably in an amount of 2 to 15 moles/mole of 1-halo-3,5-dimethyladamantane, most preferably, 3 to 6 moles/mole of 1-halo-3,5-dimethyladamantane.

Preferably, before the start of the reaction, the phosphoric acid is added dropwise at a temperature below 40°C for a period of 15 minutes. The amount of phosphoric acid is in a phosphoric acid:1-halo-3,5-dimethyladamantane mole ratio within a range of 2:1 to 4:1.

The desired product may be recovered by a variety of methods well known in the art. In one convenient method, recovery is preformed by extracting the n-acetamido-3,5-dimethyladamantane using water and n-butyl alcohol, adjusting the pH using a strong base, washing, and then concentrating the organic phase by distillation, suspending the resulting residue in acetone and water, cooling, and isolating the crystals.

The n-acetamido-3,5-dimethyladamantane prepared as described above may be converted to memantine acid addition salt form, preferably memantine hydrochloride by any method known in the art.

As discussed above, the process disclosed in U.S. Patent No. 3,391,142 involves a Ritter reaction between 1 bromo-3,5-dimethyladamantane and acetonitrile in a large amount of concentrated sulphuric acid. Due to the oxidizing power of sulphuric acid, bromine and SO₂ are by products of the reaction according to following scheme.

H₂SO₄ + 2HBr → Br₂ + SO₂ + 2H2O

In contrast, in the present invention, the use of phosphoric acid instead of sulphuric acid avoids the undesirable byproducts, while providing results that are at least as good as those obtained with processes using sulphuric acid in terms of yield, quality, and reaction time. In addition, the present invention reduces the quantity of undesirable byproducts, such that less phosphoric acid is used compared to the large amount of sulphuric acid used in the U.S. Patent No. 3,391,142, easing large scale industrial production.

Also disclosed is a crystalline form of a memantine intermediate n-acetamido-3,5-dimethyladamantane, designated herein as Form I, characterized by at least one of an x-ray powder diffraction pattern, having peaks at about 6.1,12.2, and 15.5 degrees 20, and an x-ray powder diffraction pattern substantially as depicted in Figure 1. Form I can be further characterised by x-ray powder diffraction peaks at about 12.9, 13.9, 16.8, 18.3, 19.0, 20.4, and 24.5 degrees 2θ.

In a another embodiment, the invention is directed to a process for the synthesis of memantine using n-acetamido-3,5-dimethyladamantane, and is summarized in Scheme 2.

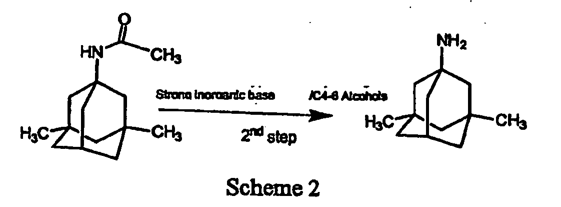

The process of the invention comprises: dissolving n-acetamido-3,5-dimethyladamantane in a solvent selected from the group consisting of C₃₋₆ alcohols in the presence of a strong inorganic base for a sufficient period of time to obtain 1-amino-3,5-dimethyladamantane; and recovering an anhydrous alcoholic solution of 1-amino-3,5-dimethyladamantane.

Preferably, dissolving n-acetamido-3,5-dimethyladamantane is by heating at 90°C to reflux, and, more preferably, to a temperature in the range of 125° to 135°C. Preferably, the solvent is n-butanol. The solvent is preferably in a volume/weight ratio of between about 2:1 and about 5:1 with respect to the n-acetamido-3,5-dimethyladamantane, most preferably about 4:1. Preferably, the strong inorganic base is an alkali metal hydroxide, most preferably KOH or NaOH. The strong inorganic base is in an amount of 4 to 12 moles/mole of n-acetamido-3,5-dimethyladamantane, most preferably 5 to 6 moles/mole of n-acetamido-3,5-dimethyladamantane. A sufficient period of time is preferably 5 to 15 hours, more preferably 9 to about 12 hours, and most preferaby 10 to 11 hours.

The desired product, 1-amino-3,5-dimethyladamantane, may be recovered by a variety of methods known in the art. In one protocol, the solution of 1-amino-3,5-dimethyladamantane is cooled, preferably to 50° to 80°C, the reaction mixture is washed with water, distilling n-butanol and water until a semisolid residue of 1-amino-3,5-dimethyladamantane (memantine free base) is obtained.

Preferably, the solution of 1-amino-3,5-dimethyladamantane is then converted into a memantine acid addition salt form, preferably memantine hydrochloride, as summarized in Scheme 3. In this process, HCl is combined with 1-amino-3,5-dimethyladamantane to obtain memantine HCl, and memantine HCl is recovered. Most preferably, the solution of 1-amino-3,5-dimethyladamantane is first concentrated under 50mm Hg vacuum [0,0667 bar] at a temperature of 55°C, to obtain a residue which is suspended in an organic solvent, such as ethyl acetate, while maintaining the temperature at about 50°C. The suspension is then cooled to 20°C for one hour, washed with water, and dried to obtain memantine HCl.

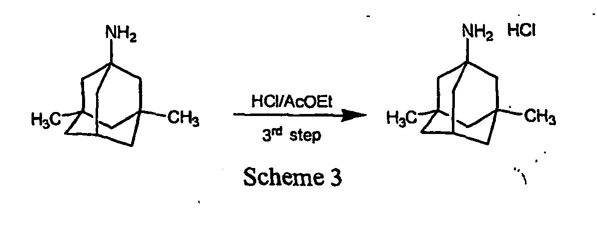

The alkaline solvolysis of the acetamido moiety, as described in U.S. Patent No. 3,391,142 and CZ 282398, requires refluxing the reactants in diethylene glycol, methanol, ethanol, or isopropyl alcohol. In contrast, in the present invention C₄₋₆ alcohols are used. In addition, as disclosed in CZ 282398, the solvolysis is carried out under pressure, while, in the process of this invention, this is unnecessary. Under such conditions, the solvolysis is completed in about 10 hours.

In a further embodiment, the invention is directed to a "one pot" process for the synthesis of memantine hydrochloride from 1-halo-3,5-dimethyladamantane without the isolation of n-acetamido-3,5-dimethyladamantane. The process of the invention comprises:
a. reacting 1-halo-3,5-dimethyladamantane and a nitrile, in the presence of phosphoric acid at 50° to 100°C and for a period of time sufficient to produce n-acetamido-3,5-dimethyladamantane,
b. dissolving n-acetamido-3,5-dimethyladamantane in a solvent selected from the group consisting of C₃₋₆ alcohols in the presence of a strong inorganic base for a sufficient period of time to obtain 1-amino-3,5-dimethyladamantane; and
c. recovering an alcoholic solution of 1-amino-3,5-dimethyladamantane.

Preferably, the solution of 1-amino-3,5-dimethyladamantane is then converted into memantine's acid addition salt form, most preferably memantine hydrochloride.

### EXAMPLES

### Example 1- Synthesis of 1-acetamido-3,5-dimethyladamantane

The following examples are provided to illustrate embodiments of the present invention.

The first step in the synthesis of 1-acetamido-3,5-dimethyladamantane, follows the following synthetic scheme.

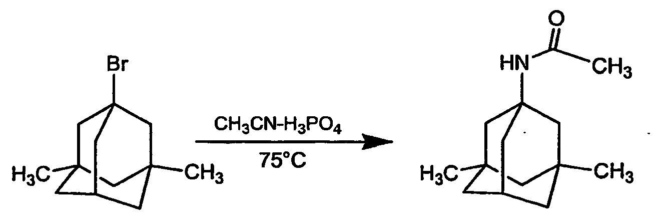

At room temperature, the 500g (2.056 mol) of 1-bromo-3,5-dimethyladamantane and 500 ml of 393 g (9.6 mol) acetonitrile were introduced into a three necked round bottom flask equipped with a thermometer, condenser, and mechanical stirrer. Then, 806g of 75% phosphoric acid (6.2 moles) were added drop wise over a period of about 15 minutes, maintaining the temperature below 40°C. The resulting biphasic mixture was heated to 87° ± 2°C, and maintained at that temperature for 3 hrs.

The 1000 ml of n-butyl alcohol and 770 ml of water were added, providing a homogeneous solution. Sodium hydroxide 30% (337.5g) was added to produce a biphasic system. The phases were separated, and 350 ml of water were added together with 30% sodium hydroxide (337.5 g) to provide an aqueous phase pH within the range of 5.5 to 7.5.

The phases were separated. The organic phase was then concentrated under a vacuum of 45 mm Hg to a residual volume of 600-650ml with an increase in the internal temperature to about 70°C. The residual suspension was cooled to 60°C, and 500 ml of acetone and 2000 ml of water were added in sequence. The resulting suspension was cooled to 0°C in one hour, and the suspension was stirred at 0°C for 1 hour. The resulting solid was filtered, and washed twice with 400 ml of water. The solid was dried overnight at 50°C. An amount of dried solid of 434.0g, corresponding to a yield of 95.4 percent was obtained with an HPLC purity of 99.8 percent.

### Example 2- Synthesis of memantine free base from n-acetyl-memantine

Ten grams (0.045 mol) of n-acetyl-memantine, 75 ml of n-butanol, and 10.5g sodium hydroxide flakes (0.263mol) is introduced into a three necked round bottom flask equipped with a thermometer, condenser, and mechanical stirrer at room temperature. The resulting suspension is heated to 120°C, and maintained at 120°C for 10 hours. The reaction mixture is cooled to 80°C, and 25 ml of water were added. The phases are separated, 25 ml of water were added to the rich organic phase, and the pH was brought to 10.5-11 with 37% HCl. After stirring, the phases are separated, and 25 ml of water were added to the rich organic phase. After stirring phases are separated again. Obtained butanolic organic phase is concentrated under vacuum at internal temperature 70°C until an oily residue (10.5g) is obtained to obtain memantine free base.

(Optionally to the oily residue AcOEt 100ml is added, and the obtained solution is decolorized with charcoal. Gaseous HCl is bubbled through the solution until acid pH. Suspension is stirred at 15°C for 1h to obtain memantine HCl

Memantine HCl is isolated by filtration, washed twice with AcOEt (10 ml each), and dried under vacuum at 55°C. MMN.HCl= 7.2g; yield: 74%)

### Example 3 - Synthesis of memantine HCl from n-acetyl memantine

Steps 2 and 3'of the synthesis of 1-amino-3,5-dimethyladamantane synthesis, memantine HCl, follow the following synthetic scheme.

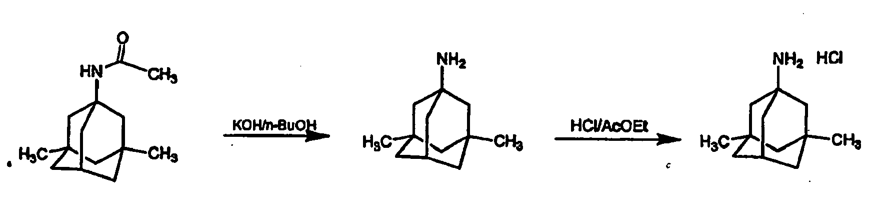

The 50 g (0.2259 mol) of n-acetyl-memantine, 200 ml of n-butanol, and 80.29 g of 89.9 percent potassium hydroxide flakes (1.288 mol) were introduced into a three necked round bottom flask equipped with a thermometer, condenser, and mechanical stirrer at room temperature. The resulting suspension was heated to 130° ± 2°C, and maintained at 130° ± 2°C for 10 to 11 hours. The reaction mixture was cooled to 50°C, and 150 ml of water were added. The phases were separated, 75 ml of water were added to the rich organic phase, and the pH is brought to 10.5-11 with 37% HCl. After stirring, the phases were separated, and 75 ml of water were added to the rich organic phase. After stirring, the phases were separated again.

The resulting solution was filtered, and 23.1 g of 37 percent HCl were added to the filtrate. The solution was concentrated under a 50 mm Hg vacuum [0,0667 bar] to a residual volume of 90ml at a temperature of 55°C. Following the vacuum concentration, 250 ml of ethyl acetate were added to the residue, while maintaining the temperature at 50°C. The suspension was cooled to 20°C, stirred at 20°C for one hour, and filtered and washed three times with 30 ml of ethyl acetate. The product was dried at 50° to 55°C for 20 hours, providing 44.2 g of product at a yield of 90.8 percent.

### Example 4 - One-pot process for the synthesis of memantine HCl

As discussed above, memantine hydrochloride may be produced in a "one pot" process.

First, 22 g (0.09042 mol) of 1-bromo-3,5-dimethyladamantane and 22 ml (0.4190 mol) of acetonitrile were introduced into a 250 ml three necked round bottom flask equipped with a thermometer, condenser, and mechanical stirrer at room temperature. Then, 41.7 g of 85 percent phosphoric acid (0.3619 mol) were added over a period of about 15 minutes through a dropping funnel, during which period the internal temperature rose to 34°C. The biphasic mixture was then warmed to 65°C. After 7 hours, a monophasic system is obtained, and the reaction was complete. The monophasic system was then cooled to room temperature, and the 55 ml of n-butanol, 55 ml of water, and 11 ml of saturated NaCl solution were added. After stirring, the phases were separated, 22 ml of water were added to organic phase, and the pH was adjusted to about 7 with 53 g of 30 percent NaOH. The phases were separated, and 22 ml of water were added to the organic phase.

After stirring, the phases were separated, and water was removed by azeotropic distillation through a plates column. Prior to distillation, the KF was 14 percent; after distillation, the Karl Fisher titration value was 0.08 percent.

Then, 49.1 g of 89.9% KOH (0.787 mol) and 100ml of n butanol were added to the anhydrified organic phase. The suspension was then warmed to 130° ± 2°C for 10 hours to provide a solution. A sample was checked using gas chromatography to determine whether the reaction had stopped. The solution was then cooled to 50°C, and 80 ml of water were added. After stirring, the phases were separated, 40 ml of water were added to the organic phase, and the pH was adjusted to 10.5 to 11 with a 37 percent solution of HCl. The phases were separated, and the organic layer was washed with 40 ml of water.

Nine grams of a 37 percent HCl solution were added to the butanolic solution. The solution was then distilled under vacuum to a residual volume of 50ml, providing a semisolid residue. Ethylacetate in an amount of 100 ml was added to residue, and the resulting suspension was stirred at 20° to 22°C for one hour, filtered, and washed with three 12 ml volumes of ethylacetate. The filtered material was dried under vacuum at 50° to 55°C for 5 to 6 hours, providing a dry weight of 16.6 g ofmemantine hydrochloride from 1-bromo-3,5-dimethyladamantane in an 86 percent yield.

While it is apparent that the invention disclosed herein is well calculated to fulfil the objects stated above, it will be appreciated that numerous modifications and embodiments may be devised by those skilled in the art.

## Claims

1. A process for the preparation of n-acetamido-3,5-dimethyladamantane, comprising:
reacting 1-halo-3,5-dimethyladamantane and a nitrile in the presence of phosphoric
acid at a temperature of 50° to 100°C for a sufficient period of time to
obtain n-acetamido-3,5-dimethyladamantane.

2. The process of claim 1, wherein the 1-halo-3,5-dimethyladamantane is 1-bromo--3,5-dimethyladamantane.

3. The process of claim 1, wherein the phosphoric acid and 1-halo-3,5-dimethyladamantane are present in a phosphoric acid:1-halo-3,5-dimethyladamantane mole ratio within a range of 2:1 to 4:1.

4. The process of claim 1, wherein reacting the temperature is 65° to 90°C.

5. The process of claim 1, wherein the sufficient period of time is 1 to 20 hours.

6. The process of claim 5, wherein the sufficient period of time is 1 to 10 hours.

7. The process of claim 1, wherein the nitrile is selected from the group consisting of propionitrile, butyronitrile, valeronitrile, phenylacetonitrile, and acetonitrile.

8. The process of claim 7, wherein the nitrile is acetonitrile.

9. The process of claim 1, wherein the nitrile is present in an amount of 2 to 15 moles/mol of1-halo-3,5-dimethyladamantane.

10. The process of claim 9, wherein the nitrile is present in an amount of 3 to 6 moles/mole of 1-halo-3,5-dimethyladamantane.

11. The process of claim 1, further comprising converting the n-acetamido-3,5-dimethyladamantane to memantine hydrochloride.

12. A process for the preparation ofmemantine hydrochloride, comprising: reacting 1-halo-3,5-dimethyladamantane and a nitrile in the presence of phosphoric acid at a temperature of 50° to 100°C for a sufficient period of time to obtain n-acetamido-3,5-dimethyladamantane; and converting the n-acetamido-3,5-dimethyladamantane to memantine hydrochloride.

13. A process for the preparation ofmemantine hydrochloride, comprising:
a) reacting 1-halo-3,5-dimethyladamantane and a nitrile in the presence of phosphoric acid at a temperature of 50° to 100°C for a sufficient period of time to obtain n-acetamido-3,5-dimethyladamantane;
b) dissolving n-acetamido-3,5-dimethyladamantane in a solvent selected from the group consisting of C₃₋₆ alcohols in the presence of a strong inorganic base for a sufficient period of time to obtain 1-amino-3,5-dimethyladamantane; and
c) reacting the 1-amino-3,5-dimethyladamantane with hydrogen chloride or hydrochloric acid to obtain memantine hydrochloride.

## Patentansprüche

1. Verfahren zur Herstellung von n-Acetamido-3,5-dimethyladamantan, umfassend:
die Durchführung einer Reaktion von 1-Halo-3,5-dimethyladamantan und einem Nitril in Anwesenheit von Phosphorsäure bei einer Temperatur von 50° bis 100° C über einen ausreichenden Zeitraum um n-Acetamido-3,5-dimethyladamantan zu erhalten.

2. Verfahren nach Anspruch 1, wobei das 1-Halogen-3,5-dimethyladamantan 1-Brom-3,5-dimethyladamantan ist.

3. Verfahren nach Anspruch 1, wobei die Phosphorsäure und 1-Halogen-3,5-dimethyladamantan in einem Molverhältnis Phosphorsäure : 1-Halogen-3,5-dimethyladamantan im Bereich von 2:1 bis 4:1 vorliegen.

4. Verfahren nach Anspruch 1, wobei bei der Durchführung der Reaktion die Temperatur zwischen 65° und 90° C beträgt.

5. Verfahren nach Anspruch 1, wobei der ausreichende Zeitraum 1 bis 20 Stunden beträgt.

6. Verfahren nach Anspruch 5, wobei der ausreichende Zeitraum 1 bis 10 Stunden beträgt.

7. Verfahren nach Anspruch 1, wobei das Nitril ausgewählt ist aus der Gruppe, bestehend aus Proprionitril, Butyronitril, Valeronitril, Phenylacetonitril und Acetonitril.

8. Verfahren nach Anspruch 7, wobei das Nitril Acetonitril ist.

9. Verfahren nach Anspruch 1, wobei das Nitril in einer Menge von 2 bis 15 Mol / Mol 1-Halogen-3,5-dimethyladamantan vorliegt.

10. Verfahren nach Anspruch 9, wobei das Nitril in einer Menge von 3 bis 6 Mol / Mol 1-Halogen-3,5-dimethyladamantan vorliegt.

11. Verfahren nach Anspruch 1, welches ferner die Umwandlung von n-Acetamido-3,5-dimethyladamantan in Memantinhydrochlorid umfasst.

12. Verfahren zur Herstellung von Memantinhydrochlorid, umfassend: die Durchführung einer Reaktion von 1-Halogen-3,5-dimethyladamantan und einem Nitril in Anwesenheit von Phosphorsäure bei einer Temperatur von 50° bis 100° C über einen ausreichenden Zeitraum um n-Acetamido-3,5-dimethyladamantan zu erhalten und die Umwandlung des n-Acetamido-3,5-dimethyladamantan in Memantinhydrochlorid.

13. Verfahren zur Herstellung von Memantinhydrochlorid, umfassend:
a) die Durchführung einer Reaktion von 1-Halogen-3,5-dimethyladamantan und einem Nitril in Anwesenheit von Phosphorsäure bei einer Temperatur von 50° bis 100° C über einen ausreichenden Zeitraum um n-Acetamido-3,5-dimethyladamantan zu erhalten.
b) das Lösen von n-Acetamido-3,5-dimethyladamantan in einem Lösungsmittel, ausgewählt aus der Gruppe, bestehend aus C₃₋₆-Alkoholen in Anwesenheit einer starken anorganischen Base über einen ausreichenden Zeitraum um 1-Amino-3,5-dimethyladamantan zu erhalten, und
c) die Durchführung einer Reaktion von 1-Amino-3,5-dimethyladamantan mit Chlorwasserstoff oder Salzsäure, um Memantinhydrochlorid zu erhalten.

## Revendications

1. Procédé pour la préparation de n-acétamido-3,5-diméthyladamantane, comprenant:
la réaction de 1-halo-3,5-diméthyladamantane avec un nitrile en présence d'acide phosphorique à une température de 50° à 100°C pendant une période de temps suffisamment longue afin d'obtenir du n-acétamido-3,5-diméthyladamantane.

2. Procédé selon la revendication 1, dans lequel le 1-halo-3,5-diméthyladamantane est le 1-bromo-3,5-diméthyladamantane.

3. Procédé selon la revendication 1, dans lequel l'acide phosphorique et le 1-halo-3,5-diméthyladamantane sont présents dans un rapport molaire acide phosphorique : 1-halo-3,5-diméthyladamantane situé dans la plage de 2:1 à 4:1.

4. Procédé selon la revendication 1, dans lequel la température de réaction est 65° à 90°C.

5. Procédé selon la revendication 1, dans lequel la période de temps suffisamment longue est 1 à 20 heures.

6. Procédé selon la revendication 5, dans lequel la période de temps suffisamment longue est 1 à 10 heures.

7. Procédé selon la revendication 1, dans lequel le nitrile est sélectionné dans le groupe se composant du propionitrile, butyronitrile, valéronitrile, phénylacétonitrile et l'acétonitrile.

8. Procédé selon la revendication 7, dans lequel le nitrile est l'acétonitrile.

9. Procédé selon la revendication 1, dans lequel le nitrile est présent à une concentration de 2 à 15 moles/mole de 1-halo-3,5-diméthyladamantane.

10. Procédé selon la revendication 9, dans lequel le nitrile est présent à une concentration de 3 à 6 moles/mole de 1-halo-3,5-diméthyladamantane.

11. Procédé selon la revendication 1, comprenant en outre la conversion du n-acétamido-3,5-diméthyladamantane en hydrochlorure de mémantine.

12. Procédé pour la préparation d'hydrochlorure de mémantine, comprenant: la réaction de 1-halo-3,5-diméthyladamantane avec un nitrile en présence d'acide phosphorique à une température de 50° à 100°C pendant une période de temps suffisamment longue afin d'obtenir du n-acétamido-3,5-diméthyladamantane ; et la conversion du n-acétamido-3,5-diméthyladamantane en hydrochlorure de mémantine..

13. Procédé pour la préparation d'hydrochlorure de mémantine, comprenant:
a) la réaction de 1-halo-3,5-diméthyladamantane avec un nitrile en présence d'acide phosphorique à une température de 50° à 100°C pendant une période de temps suffisamment longue afin d'obtenir du n-acétamido-3,5-diméthyladamantane ;
b) la dissolution de n-acétamido-3,5-diméthyladamantane dans un solvant sélectionné dans le groupe se composant d'alcools C₃₋₆ en présence d'une forte base anorganique pendant une période de temps suffisamment longue afin d'obtenir du 1-amino-3,5-diméthyladamantane ; et
c) la réaction du 1-amino-3,5-diméthyladamantane avec du chlorure d'hydrogène ou de l'acide hydrochlorique afin d'obtenir de l'hydrochlorure de mémantine.
